# DEMANDE DE BREVET EUROPEEN

(11) **EP 3 378 467 A1**
(43) Date de publication de la demande: **26.09.2018**
(21) Numéro de dépôt: 17305306.7
(22) Date de dépôt: 20.03.2017
(51) Int. Cl.: A61K 9/00, A61K 31/01, C07C 9/16

(54) **HUILE HYDROCARBONEE POUR UNE UTILISATION THERAPEUTIQUE**

(71) Demandeur: Total Marketing Services, 92800 Puteaux (FR)
(72) Inventeur: SWOBODA, Benjamin, 78630 Orgeval (FR); LEMAIRE, Philippe, 91450 Etiolles (FR)
(74) Mandataire: August Debouzy

(57) **Abrégé**

Huile hydrocarbonée destinée à être utilisée comme médicament, ladite huile hydrocarbonée comprenant une teneur en poids d'isoparaffines allant de 90 à 100%, une teneur en poids de paraffines normales allant de 0 à 10% et présentant une teneur en carbone d'origine biologique supérieure ou égale à 90 % par rapport au poids total de l'huile hydrocarbonée.

## Description

### DOMAINE DE L'INVENTION

L'invention concerne une huile hydrocarbonée classée biodégradable, d'origine biologique majoritairement isoparaffinique pour une utilisation comme médicament.

L'invention concerne également une composition comprenant une huile hydrocarbonée classée biodégradable, d'origine biologique majoritairement isoparaffinique et au moins un ammonium quaternaire pour une utilisation comme médicament.

### CONTEXTE TECHNIQUE DE L'INVENTION

Les marchés de la dermatologie ou de la pharmacie sont de plus en plus demandeurs d'ingrédient d'origine biologique pour la formulation de leurs produits. Alors que les actifs, les émulsifiants et les huiles végétales biosourcés ont été fortement développés ces dernières années et sont maintenant largement disponibles sur le marché, les principes actifs biosourcés, surtout les actifs hydrocarbonés d'origine 100% biologique, restent encore rares.

Jusqu'à présent les huiles avec un effet anti-oxydant, anti-inflammatoire ou permettant de freiner le vieillissement des cellules sont des huiles oxygénées telles que des triglycérides, des esters, des alcools, naturelles ou synthétiques. On trouve par exemple beaucoup d'huiles végétales, telles que l'huile d'olive, l'huile de Mango, l'huile d'argousier ou encore l'huile de cacao.

Cependant, toutes ces huiles ont une sensibilité importante à l'oxydation, elles ne sont pas suffisamment stables, ce qui entraine des évolutions d'odeur et de caractéristiques. L'instabilité de ces huiles végétales provient notamment de la présence, en quantité substantielle, de molécules comportant des hétéroatomes et/ou des insaturations.

Il subsiste donc le besoin de disposer de nouveaux principes actifs efficaces et présentant des caractéristiques physico-chimiques et sensorielles satisfaisantes notamment en termes de stabilité, de volatilité, d'étalement sur la peau, d'odeur, de toucher doux et de brillance, la rendant hautement compatible avec une utilisation en formulation.

La demanderesse a trouvé de manière surprenante que ce besoin peut être satisfait par l'utilisation d'une huile hydrocarbonée d'origine biologique comprenant majoritairement des isoparaffines.

La présente invention a pour objectif de fournir un principe actif médicamenteux utilisable en tant qu'agent anti-oxydant et/ou agent bloquant de radicaux libres et/ou anti-inflammatoire et/ou anti-apoptotique et/ou antibactérien et/ou antifongique.

La présente invention a pour objectif de fournir une composition pharmaceutique pour le traitement des maladies de la peau, en particulier pour diminuer l'inflammation et/ou dans le traitement des troubles de la cicatrisation.

La présente invention a également pour objectif de fournir une composition qui possède un effet anti-apoptotique amélioré.

### RÉSUMÉ DE L'INVENTION

Ces objectifs sont atteints grâce à un nouveau principe actif de type hydrocarboné pour le traitement du corps humain et/ou animal.

L'invention concerne une huile hydrocarbonée destinée à être utilisée comme médicament, ladite huile hydrocarbonée comprenant une teneur en poids d'isoparaffines allant de 90 à 100%, une teneur en poids de paraffines normales allant de 0 à 10% et présentant une teneur en carbone d'origine biologique supérieure ou égale à 90 % par rapport au poids total de l'huile hydrocarbonée.

De préférence, l'huile hydrocarbonée est destinée à être utilisée en tant qu'antioxydant et/ou agent antiradicalaire et/ou agent anti-inflammatoire et/ou anti-apoptotique et/ou antibactérien et/ou antifongique.

Selon un mode de réalisation de l'invention, l'huile hydrocarbonée est destinée à être utilisée dans le traitement des maladies de la peau telles que le psoriasis, les verrues et les cors, l'acné, les troubles de la séborrhée, l'herpès, les mycoses, l'eczéma, le zona et/ou des troubles de la cicatrisation.

Selon un mode de réalisation de l'invention, l'huile hydrocarbonée est destinée à être utilisée pour une application topique.

Selon un autre mode de réalisation de l'invention, l'huile hydrocarbonée est destinée à être utilisée pour une administration par voie systémique.

Selon un mode de réalisation, l'huile hydrocarbonée comprend une teneur en poids d'isoparaffines allant de 95% à 100% et préférentiellement de 98% à 100% par rapport au poids total de l'huile hydrocarbonée.

Selon un mode de réalisation, l'huile hydrocarbonée est choisie parmi les isoparaffines non-cycliques comprenant de 14 à 18 atomes de carbone.

Selon un mode de réalisation, l'huile hydrocarbonée comprend :
- une teneur en poids de paraffines normales inférieure ou égale à 5% et préférentiellement inférieure ou égale à 2% par rapport au poids total de l'huile hydrocarbonée ; et/ou
- une teneur en poids de composés naphténiques inférieure ou égale à 1%, de préférence inférieure ou égale à 0,5% et préférentiellement inférieure ou égale à 100 ppm par rapport au poids total de l'huile hydrocarbonée ; et/ou
- une teneur en poids de composés aromatiques inférieure ou égale à 500 ppm, de préférence inférieure ou égale à 300 ppm, préférentiellement inférieure ou égale à 100 ppm, plus préférentiellement inférieure ou égale à 50 ppm et avantageusement inférieure ou égale à 20 ppm par rapport au poids total de l'huile hydrocarbonée.

Selon un mode de réalisation, l'huile hydrocarbonée a:
- une température d'ébullition allant de 230 à 340°C, de préférence de 235 à 330°C et plus préférentiellement de 240 à 325°C selon la norme ASTM D86 ; et/ou
- un point éclair supérieur ou égal à 110°C selon la norme EN ISO 2719 ; et/ou
- une pression de vapeur à 20°C inférieure ou égale à 0,01kPa.

Selon un mode de réalisation, l'huile hydrocarbonée est obtenue par un procédé d'hydrogénation catalytique à une température de 80 à 180°C et à une pression de 50 à 160 bars d'une charge d'origine biologique désoxygénée et/ou isomérisée.

Selon un mode de réalisation, l'huile hydrocarbonée se présente sous la forme d'une composition pharmaceutique comprenant au moins une huile hydrocarbonée telle que définie dans l'invention, de préférence en une quantité allant de 0,5 à 80%, préférentiellement de 1 à 50% et avantageusement de 5 à 30% en poids par rapport au poids total de la composition pharmaceutique. Selon un mode de réalisation, la composition pharmaceutique comprend :
- au moins un corps gras choisi parmi : les huiles végétales, les huiles hydrocarbonées autres que l'huile hydrocarbonée définie dans les revendications 1 à 10, les beurres végétaux, les éthers et alcools gras, les esters huileux, les alcanes et les huiles silicones et/ou
- au moins un additif.

Selon un mode de réalisation, la composition pharmaceutique est une composition anhydre, une émulsion telle qu'une émulsion eau-dans-huile (E/H), une émulsion huile-dans-eau (H/E) ou une émulsion multiple (notamment E/H/E ou H/E/H), une nano-émulsion, ou encore une dispersion ou un gel.

Selon un autre mode de réalisation, l'huile hydrocarbonée se présente sous la forme d'un alicament comprenant au moins une huile hydrocarbonée telle que définie dans l'invention, de préférence en une quantité allant de 0,5 à 80%, préférentiellement de 1 à 50% et avantageusement de 5 à 30% en poids par rapport au poids total de l'alicament.

Selon un mode de réalisation de l'invention, l'huile hydrocarbonée destinée à être utilisée selon l'invention est en combinaison avec un ammonium quaternaire.

De préférence, l'ammonium quaternaire est choisi parmi le chlorure de benzalkonium et le chlorure de didécyldiméthylammonium, seuls ou en mélange.

Selon un mode de réalisation, l'ammonium quaternaire représente de 0,1 ppm à 40% en poids, de préférence de 0,5 ppm à 30% en poids, préférentiellement de 1 ppm à 20% en poids, encore plus préférentiellement de 3 ppm à 10% en poids, idéalement de 5 ppm à 1% en poids du poids total de la combinaison huile hydrocarbonée et ammonium quaternaire ou de la composition pharmaceutique ou de l'alicament.

### DESCRIPTION DETAILLÉE DE L'INVENTION

L'invention concerne une huile hydrocarbonée destinée à être utilisée comme médicament, de préférence à titre de principe actif, ladite huile hydrocarbonée comprenant une teneur en poids d'isoparaffines allant de 90 à 100%, une teneur en poids de paraffines normales allant de 0 à 10% et une teneur en carbone d'origine biologique supérieure à 95% par rapport au poids total de l'huile hydrocarbonée.

Ainsi, l'huile hydrocarbonée définie dans la présente invention peut être utilisée pour le traitement, en particulier thérapeutique, du corps humain et/ou animal.

L'huile hydrocarbonée définie dans la présente invention peut en particulier être utilisée comme agent anti-oxydant, agent bloquant de radicaux libres et/ou agent anti-inflammatoire et/ou agent anti-apoptotique.

L'huile hydrocarbonée utilisée selon l'invention permet de disposer d'une composition pharmaceutique classée non-irritante, biodégradable et non-odorante.

L'huile hydrocarbonée utilisée selon l'invention permet en particulier d'obtenir des compositions pharmaceutiques stables, notamment grâce à l'huile hydrocarbonée définie dans la présente invention qui comprend de faibles proportions, voire qui est substantiellement ou totalement exempte, de composés insaturés et/ou de composés comprenant des hétéroatomes.

A titre préliminaire on notera que, dans la description et les revendications suivantes, l'expression « compris entre » doit s'entendre comme incluant les bornes citées.

### Huile hydrocarbonée :

L'huile hydrocarbonée utilisée selon l'invention comprend de préférence une teneur en poids de composés isoparaffiniques supérieure ou égale à 90%, préférentiellement supérieure ou égale à 95 % et avantageusement supérieure ou égale à 98% par rapport au poids total de l'huile hydrocarbonée.

Selon un mode de réalisation, les composés isoparaffiniques présents dans l'huile hydrocarbonée utilisée selon l'invention comportent de 12 à 30 atomes de carbone, de préférence de 13 à 19 atomes de carbone, de préférence encore de 14 à 18 atomes de carbone.

L'huile hydrocarbonée utilisée selon l'invention comprend de préférence une teneur en poids de paraffines normales inférieure ou égale à 10%, préférentiellement inférieure ou égale à 5 % et avantageusement inférieure ou égale à 2%.

L'huile hydrocarbonée utilisée selon l'invention comprend avantageusement une majorité d'isoparaffines et une minorité de paraffines normales. Ces isoparaffines sont avantageusement des isoparaffines non-cycliques. De préférence, l'huile hydrocarbonée a un ratio massique d'isoparaffines sur paraffines normales d'au moins 12 :1, préférentiellement de 15 :1 et plus préférentiellement de 20 :1. De manière encore plus avantageuse l'huile hydrocarbonée utilisée selon l'invention ne contient pas de paraffines normales.

Selon un mode de réalisation, l'huile hydrocarbonée utilisée selon l'invention comprend de préférence une teneur en poids d'isoparaffines allant de 90 à 100% et une teneur en paraffines normales allant de 0 à 10%, préférentiellement de 95% à 100% d'isoparaffines et de 0 à 5% de paraffines normales et plus préférentiellement de 98% à 100% d'isoparaffines et de 0 à 2 % de paraffines normales.

Selon un mode de réalisation, l'huile hydrocarbonée utilisée selon l'invention comprend de préférence une teneur en poids d'isoparaffines allant de 90% à 100% et une teneur en paraffines normales allant de 0 à 10%, préférentiellement de 95% à 100% d'isoparaffines choisies parmi les alcanes comportant de 12 à 30 atomes de carbone, de préférence de 14 à 18 atomes de carbone, de préférence encore de 14 à 17 atomes de carbone.

Selon un mode de réalisation, l'huile hydrocarbonée utilisée selon l'invention comprend :
- des isoparaffines ayant 15 atomes de carbone et des isoparaffines ayant 16 atomes de carbone en une quantité combinée allant de 80 à 98% en poids, par rapport au poids total de l'huile hydrocarbonée, ou
- des isoparaffines ayant 16 atomes de carbone, des isoparaffines ayant 17 atomes de carbone et des isoparaffines ayant 18 atomes de carbone en une quantité combinée allant de 80 à 98% en poids, par rapport au poids total de l'huile hydrocarbonée, ou
- des isoparaffines ayant 17 atomes de carbone et des isoparaffines ayant 18 atomes de carbone en une quantité combinée allant de 80 à 98% en poids, par rapport au poids total de l'huile hydrocarbonée.

L'huile hydrocarbonée utilisée selon l'invention comprend de préférence une teneur en poids de composés naphténiques inférieure ou égale à 1%, préférentiellement inférieure ou égale à 0.5% et plus préférentiellement inférieure ou égale à 100 ppm.

Selon un autre mode de réalisation préféré, l'huile hydrocarbonée utilisée selon l'invention comprend une teneur en poids d'isoparaffines allant de 90 à 100%, une teneur en poids de paraffines normales allant de 0 à 10% et une teneur en poids de naphtènes inférieure ou égale à 1%. Préférentiellement l'huile hydrocarbonée comprend une teneur en poids allant de 95% à 100% d'isoparaffines, de 0 à 5% de paraffines normales et une teneur en poids de naphtènes inférieure ou égale à 0,5%. Plus préférentiellement elle comprend une teneur en poids allant de 98% à 100% d'isoparaffines, de 0 à 2 % de paraffines normales et une teneur en poids de naphtènes inférieure ou égale à 100 ppm.

L'huile hydrocarbonée mise en oeuvre selon l'invention est avantageusement exempte de composés aromatiques. Par exempte, on entend une teneur en poids de composés aromatiques inférieure ou égale à 500 ppm, de préférence inférieure ou égale à 300 ppm, préférentiellement inférieure ou égale à 100 ppm, plus préférentiellement inférieure ou égale à 50 ppm et avantageusement inférieure ou égale à 20 ppm mesurée par exemple par spectrométrie UV.

La teneur en poids en isoparaffines, en n-paraffines, en naphtènes et/ou en aromatiques de l'huile hydrocarbonée peut être déterminée selon des méthodes bien connues de l'homme du métier. On peut citer à titre d'exemple non limitatif, une méthode par chromatographie en phase gazeuse.

Selon un autre mode de réalisation préféré, l'huile hydrocarbonée utilisée selon l'invention comprend une teneur en poids d'isoparaffines allant de 90 à 100%, une teneur en poids de paraffines normales allant de 0 à 10%, une teneur en poids de naphtènes inférieure ou égale à 1% et une teneur en poids de composés aromatiques inférieure ou égale à 500 ppm. Préférentiellement l'huile hydrocarbonée comprend une teneur en poids allant de 95% à 100% d'isoparaffines, de 0 à 5% de paraffines normales, une teneur en poids de naphtènes inférieure ou égale à 0,5% et une teneur en poids de composés aromatiques inférieure ou égale à 300 ppm, de préférence inférieure à 100 ppm, préférentiellement inférieure à 50 ppm, plus préférentiellement inférieure à 20 ppm. Préférentiellement aussi l'huile hydrocarbonée comprend une teneur en poids allant de 95% à 100% d'isoparaffines, de 0 à 5% de paraffines normales et une teneur en poids de composés aromatiques inférieure ou égale à 100 ppm. Plus préférentiellement elle comprend une teneur en poids allant de 98% à 100% d'isoparaffines, de 0 à 2 % de paraffines normales, une teneur en poids de naphtènes inférieure ou égale à 100 ppm et une teneur en poids de composés aromatiques inférieure ou égale à 100 ppm.

Selon un mode de réalisation de l'invention, l'huile hydrocarbonée comprend une teneur en carbone d'origine biologique supérieure ou égale à 95%, de préférence supérieure ou égale à 98% et préférentiellement de 100%.

L'huile hydrocarbonée mise en oeuvre selon l'invention a également de préférence une teneur en poids de composés soufrés extrêmement basse, typiquement inférieure ou égale à 5 ppm, préférentiellement inférieure ou égale à 3 ppm et plus préférentiellement inférieure ou égale à 0,5 ppm à un niveau trop bas pour être détectée grâce à des analyseurs de basse-teneur en soufre conventionnels.

L'huile hydrocarbonée mise en oeuvre selon l'invention a également de préférence un point éclair supérieur ou égal à 110°C, préférentiellement supérieur ou égal à 120°C et plus préférentiellement supérieur ou égal à 140°C selon la norme EN ISO 2719. Un point éclair élevé, typiquement supérieure à 110°C permettant entre autre de pallier d'une part les problèmes de sécurité lors du stockage et du transport en évitant une inflammabilité trop sensible de l'huile hydrocarbonée.

L'huile hydrocarbonée a aussi de préférence une pression de vapeur à 20°C inférieure ou égale à 0,01kPa.

Selon un mode de réalisation, l'huile hydrocarbonée mise en oeuvre selon l'invention a également de préférence un point éclair supérieur ou égal à 110°C selon la norme EN ISO 2719 et une pression de vapeur à 20°C inférieure ou égale à 0,01kPa. Préférentiellement l'huile hydrocarbonée a un point éclair supérieur ou égal supérieur ou égal à 120°C et une pression de vapeur à 20°C inférieure ou égale à 0,01kPa. Et plus préférentiellement, elle a un point éclair supérieur ou égal à 130°C et une pression de vapeur à 20°C inférieure ou égale à 0,01kPa.

L'huile hydrocarbonée mise en oeuvre selon l'invention présente des températures d'ébullition, un point éclair et une pression de vapeur permettant de pallier les problèmes d'inflammabilité, d'odeur et de volatilité.

L'huile hydrocarbonée selon l'invention a en outre de préférence une viscosité cinématique à 40°C inférieure ou égale à 5 cSt, préférentiellement inférieure ou égale à 4 cSt et plus préférentiellement inférieure ou égale à 3 cSt selon la norme EN ISO 3104.

### Procédé d'obtention :

De telles compositions d'huiles hydrocarbonées peuvent être obtenues de la façon suivante. L'huile hydrocarbonée utilisée selon l'invention est une coupe hydrocarbonée qui est issue de la conversion de la biomasse.

Par issue de la conversion de la biomasse, on entend une coupe hydrocarbonée produite à partir de matières premières d'origine biologique.

De préférence, la coupe hydrocarbonée d'origine biologique est obtenue par un procédé comprenant des étapes d'hydrodésoxygénation (HDO) et d'isomérisation (ISO). L'étape d'hydrodésoxygénation (HDO) conduit à la décomposition des structures des esters biologiques ou des constituants triglycérides, à l'élimination des composés oxygénés, phosphorés et soufrés et à l'hydrogénation des liaisons oléfiniques. Le produit issu de la réaction d'hydrodésoxygénation est ensuite isomérisé. Une étape de fractionnement peut de préférence suivre les étapes d'hydrodésoxygénation et d'isomérisation. De manière avantageuse, les fractions d'intérêt sont ensuite soumises à des étapes d'hydrotraitement puis de distillation afin obtenir les spécifications de l'huile hydrocarbonée souhaitée selon l'invention.

Ce procédé HDO/ISO est mis en oeuvre sur une charge biologique brute, encore appelée biomasse ou matière première d'origine biologique, sélectionnée dans le groupe consistant en des huiles végétales, des graisses animales, des huiles de poisson et leur mélange. Les matières premières d'origine biologique appropriées sont par exemple l'huile de colza, l'huile de canola, le talloil, l'huile de tournesol, l'huile de soja, l'huile de chanvre, l'huile d'olive, l'huile de lin, l'huile de moutarde, l'huile de palme, l'huile d'arachide, l'huile de ricin, l'huile de noix de coco, les graisses animales telles que le suif, les graisses alimentaires recyclées, les matières premières issues du génie génétique, et les matières premières biologiques produites à partir de microorganismes tels que les algues et les bactéries. Des produits de condensation, esters ou autres dérivés obtenus à partir de matériaux biologiques bruts peuvent également servir de matières premières.

De préférence, la matière première d'origine biologique est un ester ou un dérivé triglycéride. Ce matériaux est soumis tout d'abord à une étape d'hydrodésoxygénation (HDO) pour décomposer la structure des esters ou triglycérides constitutifs et éliminer les composés oxygénés, phosphorés et soufrés de manière concomitante à l'hydrogénation des liaisons oléfiniques. Cette étape d'hydrodésoxygénation (HDO) de la matière première d'origine biologique est suivie par une isomérisation du produit ainsi obtenu conduisant à la ramification de la chaîne hydrocarbonée et à une amélioration des propriétés de la paraffine à basses températures.

Durant l'étape HDO, l'hydrogène et la matière première d'origine biologique sont passés sur un lit catalytique d'hydrodésoxygénation de manière simultanée ou à contre-courant. Durant l'étape HDO, la pression et la température sont comprises entre 20 et 150 bars et entre 200 et 500°C respectivement. Des catalyseurs classiques et connus d'hydrodésoxygénation sont utilisés durant cette étape. Éventuellement, la matière première d'origine biologique peut être soumise à une pré-hydrogénation sous conditions douces pour éviter les réactions secondaires des doubles liaisons avant l'étape HDO. Après l'étape d'hydrodésoxygénation, le produit issu de la réaction est soumis à une étape d'isomérisation (ISO) où l'hydrogène et le produit, et éventuellement un mélange de n-paraffines, sont passés sur des lits catalytiques d'isomérisation de manière simultanée ou à contre-courant. Lors de l'étape ISO, la pression et la température sont comprises entre 20 et 150 bars et entre 200 et 500°C respectivement. Des catalyseurs classiques et connus d'isomérisation sont utilisés durant cette étape.

Des procédés secondaires additionnels peuvent également être mise en oeuvre (comme des mélanges intermédiaires, des piégeages ou autres procédés de la sorte).

Le produit issu des étapes HDO/ISO peut éventuellement être fractionné afin d'obtenir les coupes d'intérêt.

Divers procédés HDO/ISO sont décrits dans la littérature. La demande WO2014/033762 décrit un procédé comprenant une étape de pré-hydrogénation, une étape d'hydrodésoxygénation (HDO) et une étape d'isomérisation opérées à contre-courant. La demande de brevet EP1728844 décrit un procédé de production de composés hydrocarbonés à partir d'un mélange de composés d'origine végétale et animale. Ce procédé comprend une étape de prétraitement du mélange permettant d'enlever les contaminants, comme par exemple les sels de métaux alcalins, suivie d'une étape d'hydrodésoxygénation (HDO) et d'une étape d'isomérisation. La demande de brevet EP2084245 décrit un procédé de production d'un mélange hydrocarboné qui peut être utilisé comme gazole ou dans une composition de gazole par hydrodésoxygénation d'un mélange d'origine biologique contenant des esters d'acides gras éventuellement en mélange avec des acides gras libres, par exemple des huiles végétales comme l'huile de tournesol, l'huile de colza, l'huile de canola, l'huile de palme ou l'huile de pin, suivi d'une hydroisomérisation sur des catalyseurs spécifiques. La demande de brevet EP2368967 décrit un tel procédé et le produit obtenu par ce procédé. La demande WO2016/185046 décrit un procédé d'obtention d'une huile hydrocarbonée utilisée selon l'invention, dans lequel l'huile hydrocarbonée est obtenue par un procédé d'hydrogénation catalytique à une température de 80 à 180°C et à une pression de 50 à 160 bars d'une charge d'origine biologique désoxygénée et isomérisée.

Avantageusement, la matière première d'origine biologique contient moins de 15 ppm en poids de soufre, de préférence moins de 8 ppm, préférentiellement moins de 5 ppm et plus préférentiellement moins de 1 ppm selon la norme EN ISO 20846. Idéalement la charge ne comprend pas de soufre en tant que matière première d'origine biosourcée.

Avant l'étape d'hydrotraitement, une étape de préfractionnement peut avoir lieu. Une coupe plus étroite en entrée d'unité d'hydrogénation permet d'obtenir une coupe étroite en sortie d'unité. En effet, les points d'ébullition de coupes préfractionnées sont compris entre 220 et 330 °C tandis que les coupes qui n'ont pas été préfractionnées ont typiquement des points d'ébullition comprise entre 150 et 360°C.

La charge désoxygénée et isomérisée issue du procédé HDO/ISO est ensuite hydrogénée.

L'hydrogène utilisé dans l'unité d'hydrogénation est typiquement de l'hydrogène hautement purifié. On entend par hautement purifié, de l'hydrogène d'une pureté par exemple supérieure à 99%, même si d'autres grades peuvent également être utilisés.

L'étape d'hydrogénation est effectuée grâce à des catalyseurs. Les catalyseurs d'hydrogénation types peuvent être soit massiques soit supportés et peuvent comprendre les métaux suivants : nickel, platine, palladium, rhénium, rhodium, tungstate de nickel, nickel-molybdène, molybdène, cobalt-molybdène. Les supports peuvent être de la silice, de l'alumine, de la silice-alumine ou des zéolithes.

Un catalyseur préféré est un catalyseur à base de nickel sur support d'alumine dont l'aire de surface spécifique varie entre 100 et 200 m²/g de catalyseur ou un catalyseur massique à base de nickel. Les conditions d'hydrogénation sont typiquement les suivantes :
- Pression : 50 à 160 bars, de préférence 80 à 150 bars et plus préférentiellement 90 à 120 bars ;
- Température : 80 à 180 °C, de préférence 120 à 160 °C et plus préférentiellement 150 à 160 °C ;
- Vitesse volumique horaire (VVH): 0,2 à 5 hr⁻¹, de préférence 0,4 à 3 hr⁻¹ et plus préférentiellement 0,5 à 0,8 hr⁻¹;
- Taux de traitement par l'hydrogène : adapté aux conditions mentionnées ci-dessus et pouvant aller jusqu'à 200 Nm³/tonnes de charge à traiter.

La température dans les réacteurs est typiquement comprise entre 150 et 160°C avec une pression d'environ 100 bars tandis que la vitesse volumique horaire est d'environ 0,6 hr⁻¹ avec un taux de traitement adapté en fonction de la qualité de la charge à traiter et des paramètres du premier réacteur d'hydrogénation.

L'hydrogénation peut avoir lieu dans un ou plusieurs réacteurs en série. Les réacteurs peuvent comprendre un ou plusieurs lits catalytiques. Les lits catalytiques sont généralement des lits catalytiques fixes.

Le procédé d'hydrogénation comprend de préférence deux ou trois réacteurs, de préférence trois réacteurs et est plus préférentiellement réalisé dans trois réacteurs en série.

Le premier réacteur permet le piégeage des composés soufrés et l'hydrogénation d'essentiellement tous les composés insaturés et jusqu'à environ 90 % des composés aromatiques. Le produit issu du premier réacteur ne contient substantiellement aucun composé soufré. Au second stade c'est-à-dire dans le second réacteur, l'hydrogénation des aromatiques se poursuit et jusqu'à 99 % des aromatiques sont de ce fait hydrogénés.

Le troisième stade dans le troisième réacteur est un stade de finition permettant d'obtenir des teneurs en aromatiques inférieures ou égales à 500 ppm, de préférence inférieures ou égales à 300 ppm, préférentiellement inférieures ou égales à 100 ppm, plus préférentiellement inférieures ou égales à 50 ppm, et idéalement inférieures ou égales à 20 ppm même dans le cas de produits à haut point d'ébullition par exemple supérieur à 300°C.

Il est possible d'utiliser un réacteur qui comporte deux ou trois lits catalytiques ou plus. Les catalyseurs peuvent être présents à des quantités variables ou essentiellement égales dans chaque réacteur ; pour trois réacteurs, les quantités en fonction du poids peuvent par exemple être de 0,05-0,5/0,10-0,70/0,25-0,85, de préférence 0,07-0,25/0,15-0,35/0,4-0,78 et plus préférentiellement de 0,10-0,20/0,20-0,32/0,48-0,70.

Il est également possible d'utiliser un ou deux réacteurs d'hydrogénation au lieu de trois.

Il est également possible que le premier réacteur soit composé de réacteurs jumeaux mis en oeuvre de manière alternative. Ce mode d'opérabilité permet notamment un chargement et un déchargement facilité des catalyseurs : lorsque le premier réacteur comprend le catalyseur saturé en premier (substantiellement tout le soufre est piégé sur et/ou dans le catalyseur) il doit être changé souvent.

Un seul réacteur peut également être utilisé dans lequel deux, trois lits catalytiques ou plus sont installés.

Il peut être nécessaire d'insérer des boîtes de quench (au sens anglais « d'étouffement de la réaction ») dans le système de recycle ou entre les réacteurs pour refroidir les effluents d'un réacteur à un autre ou d'un lit catalytique à un autre afin de contrôler les températures et l'équilibre hydrothermique de chaque réaction. Selon un mode de réalisation préféré, il n'y a pas d'intermédiaires de refroidissement ou d'étouffement.

Selon un mode de réalisation, le produit issu du procédé et/ou les gaz séparés sont au moins en partie recyclé(s) dans le système d'alimentation des réacteurs d'hydrogénation. Cette dilution contribue à maintenir l'exothermicité de la réaction dans des limites contrôlées, en particulier au premier stade. Le recyclage permet en outre un échange de chaleur avant la réaction et aussi un meilleur contrôle de la température.

L'effluent de l'unité d'hydrogénation contient principalement le produit hydrogéné et de l'hydrogène. Des séparateurs flash sont utilisés pour séparer les effluents en phase gazeuse, principalement l'hydrogène résiduel, et en phase liquide, principalement les coupes hydrocarbonées hydrogénés. Le procédé peut être effectué en utilisant trois séparateurs flash, un à pression élevée, un à pression intermédiaire et un à basse pression très proche de la pression atmosphérique.

L'hydrogène gazeux qui est recueilli en haut des séparateurs flash peut être recyclé dans le système d'alimentation de l'unité d'hydrogénation ou à différents niveaux dans les unités d'hydrogénation entre les réacteurs.

Selon un mode de réalisation, le produit final est séparé à pression atmosphérique. Il alimente ensuite directement une unité de fractionnement sous vide. De préférence, le fractionnement se fera à une pression comprise entre 10 et 50 mbars et plus préférentiellement à environ 30 mbars.

Le fractionnement peut être effectué de façon à ce qu'il soit possible de retirer simultanément divers fluides hydrocarbonés de la colonne de fractionnement et à ce que leur température d'ébullition puisse être prédéterminée.

En adaptant la charge au travers de ses points d'ébullition initiaux et finaux, les réacteurs d'hydrogénation, les séparateurs et l'unité de fractionnement peuvent donc être directement connectés sans qu'il soit nécessaire d'utiliser des cuves intermédiaires. Cette intégration de l'hydrogénation et du fractionnement permet une intégration thermique optimisée associée à une réduction du nombre d'appareils et à une économie d'énergie.

L'huile hydrocarbonée mise en oeuvre dans l'invention est avantageusement une coupe hydrocarbonée ayant de préférence un intervalle de distillation ID (en °C) allant de 230°C à 340°C, de préférence de 235°C à 330°C et plus préférentiellement de 240°C à 325°C, encore plus préférentiellement de 240°C à 290°C ou encore de 240°C à 270°C mesuré selon la norme ASTM D86. De préférence, la différence entre le point d'ébullition initial et le point d'ébullition final est inférieure ou égale à 80°C, préférentiellement inférieure ou égale à 70°C, plus préférentiellement inférieure ou égale à 60°C et avantageusement comprise entre 40 et 50°C. L'huile hydrocarbonée peut comprendre une ou plusieurs fractions d'intervalles de distillation compris dans les intervalles décrits ci-dessus.

Avantageusement, l'huile hydrocarbonée mise en oeuvre dans l'invention est totalement saturée. De préférence, les composants de l'huile hydrocarbonée sont choisis parmi les isoparaffines comprenant 12 à 30 atomes de carbone, préférentiellement 13 à 19 atomes de carbone et plus préférentiellement 14 à 18 atomes de carbones.

L'huile hydrocarbonée utilisée selon l'invention comprend avantageusement une teneur en isohexadécane inférieure ou égale à 50%.

L'huile hydrocarbonée utilisée selon l'invention est idéalement issue du traitement de matières premières d'origine biologique. Le terme de «bio-carbone" indique que le carbone est d'origine naturelle et vient d'un biomatériau, comme indiqué ci-après. La teneur en bio-carbone et la teneur en biomatériau sont des expressions indiquant la même valeur. Un matériau d'origine renouvelable ou biomatériau est un matériau organique dans lequel le carbone est issu du CO₂ fixé récemment (sur une échelle humaine) par photosynthèse à partir de l'atmosphère. Un biomatériau (Carbone 100% d'origine naturelle) présente un rapport isotopique ¹⁴C /¹²C supérieur à 10⁻¹², typiquement environ 1,2 x 10⁻¹², tandis qu'un matériau fossile a un rapport nul. En effet, le ¹⁴C isotopique est formé dans l'atmosphère et est alors intégré par photosynthèse, selon une échelle de temps de quelques des dizaines d'années au maximum. La demi-vie du ¹⁴C est 5730 années. Ainsi, les matériaux issus de la photosynthèse, à savoir les plantes d'une manière générale, ont nécessairement un contenu maximum en isotope ¹⁴C.

La détermination de la teneur en biomatériau ou en bio-carbone est donnée conformément aux normes ASTM D 6866-12, la méthode B (ASTM D 6866-06) et ASTM D 7026 (ASTM D 7026-04). L'huile hydrocarbonée utilisée selon l'invention présente une teneur en biomatériau d'au moins 90%. Cette teneur est avantageusement plus élevée, en particulier supérieure ou égale à 95%, préférablement supérieure ou égale à 98 % et avantageusement égale à 100%.

En plus d'une teneur particulièrement élevée en biomatériau, l'huile hydrocarbonée utilisée selon l'invention possède une biodégradabilité particulièrement bonne. La biodégradation d'un produit chimique organique se réfère à la réduction de la complexité des composés chimiques grâce à l'activité métabolique de micro-organismes. Dans des conditions aérobies, les micro-organismes transforment les substances organiques en dioxyde de carbone, eau et biomasse. La méthode OCDE 306, est utilisée pour l'évaluation de la biodégradabilité des substances individuelles dans l'eau de mer. Selon cette méthode, l'huile hydrocarbonée a une biodégradabilité à 28 jours d'au moins 60%, de préférence d'au moins 70%, plus préférablement d'au moins 75% et avantageusement d'au moins 80%.

### Utilisation de l'huile hydrocarbonée

Les inventeurs ont découvert que l'huile hydrocarbonée telle que définie ci-dessus peut être utilisée comme agent antioxydant et/ou agent antiradicalaire. En effet, de manière surprenante, il a été découvert que l'huile hydrocarbonée définie dans la présente invention permettait de bloquer les radicaux libres.

En outre, l'huile hydrocarbonée peut être utilisée en tant que principe actif d'un médicament pour le traitement du corps humain ou animal.

L'huile hydrocarbonée a en particulier une action anti-oxydante, antiradicalaire, anti-inflammatoire, anti-dégénérescence cellulaire ou anti-apoptose, antibactérien, et/ou antifongique.

L'huile hydrocarbonée permet notamment de diminuer l'activation du récepteur P2X7.

L'huile hydrocarbonée utilisée selon l'invention permet également de bloquer l'effet apoptotique d'une molécule, telle que le chlorure de benzalkonium.

L'huile hydrocarbonée définie dans la présente invention pourrait ainsi avantageusement être utilisée dans le traitement des maladies de la peau et/ou du cuir chevelu et/ou des troubles de la cicatrisation.

Selon un mode de réalisation, les maladies de la peau et/ou du cuir chevelu sont choisies parmi le psoriasis, les verrues et les cors, l'acné, les troubles de la séborrhée, l'herpès, les mycoses, l'eczéma, le zona.

L'huile hydrocarbonée utilisée comme médicament selon l'invention peut être administrée par voie orale, par voie entérale ou parentérale ou par voie locale en application topique sur la peau, le cuir chevelu, les cheveux et les muqueuses ou par injection par exemple par voie sous cutanée.

L'huile hydrocarbonée peut être utilisée seule ou sous la forme d'une composition pharmaceutique comprenant l'huile hydrocarbonée, de préférence en une quantité allant de 0,5 à 80%, préférentiellement de 1 à 50% et avantageusement de 5 à 30% en poids par rapport au poids total de la composition.

L'huile hydrocarbonée peut être appliquée par voie topique, par exemple sur la peau du visage ou du corps, sur le cuir chevelu ou les cheveux. Plus particulièrement, l'huile hydrocarbonée définie dans la présente invention peut être appliquée sur une plaie ou une inflammation de la peau ou du cuir chevelu pour accélérer et/ou améliorer la cicatrisation.

L'huile hydrocarbonée utilisée selon l'invention présente une très bonne miscibilité avec les autres corps gras classiquement utilisés dans les domaines pharmaceutiques. En particulier, l'huile hydrocarbonée utilisée selon l'invention peut comprendre en outre au moins un corps gras choisi parmi : les huiles végétales, les huiles hydrocarbonées (autres que l'huile hydrocarbonée utilisée selon l'invention et définie ci-dessus), les beurres végétaux, les éthers et alcools gras, les esters huileux et alcanes et les huiles silicones et/ou au moins un additif.

Les huiles hydrocarbonées sont généralement des corps gras issus de procédés pétrochimiques. A titre d'exemple, on peut citer, les huiles minérales, les isoparaffines, les cires, les paraffines, les polyisobutènes ou encore les polydécènes.

Des exemples d'huiles végétales sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ou de camélia.

Les beurres végétaux sont des corps gras qui ont les mêmes propriétés que les huiles végétales. La différence entre les deux consiste dans le fait que les beurres se présentent sous forme solide à température ambiante. Aussi, contrairement aux huiles végétales, la matière première dont est extrait un beurre (pulpe, graines ou amandes) est chauffée après avoir été broyée pour l'extraction de la matière grasse. Comme les huiles végétales, les beurres peuvent être raffinés pour assurer une meilleure conservation, neutraliser les odeurs, améliorer la couleur et la consistance. Riches en antioxydants et nourrissants les propriétés cosmétiques des beurres végétaux améliorent l'élasticité de la peau, protègent des agressions extérieures en laissant un film protecteur sur l'épiderme et réduisant ainsi la déshydratation, réparent et apaisent en régénérant le film hydrolipidique naturel de la peau. Des exemples de beurres végétaux sont notamment le beurre de karité, le beurre de cacao, le beurre de mangue, le beurre de shorea ou encore le beurre d'olive.

Les éthers et les alcools gras sont des substances cireuses grasses à longue chaîne et aux propriétés remarquables notamment filmogènes, émollientes, hydratantes, adoucissantes et protectrices. Ils agissent comme huiles hydratantes et comme émulsifiants. Des exemples d'alcool gras ou d'éthers sont : le cetyl Alcohol, le Stearyl Alcohol, le myristyl alcohol, le lauryl alcohol, le behenyl alcohol, le cetearyl alcohol, le dicaprylyl ethers, les stearyl ethers ou l'octyldodecanol (identifiés par leur dénomination INCI).

Les esters huileux ou huiles estérifiées sont le produit d'une réaction entre des acides gras (acides à chaînes plus longues, comme par exemple l'acide stéarique, l'acide oléique, l'acide palmitique) et des alcools (des alcools gras ou des polyols comme le glycérol). Ces huiles peuvent contenir des substances issues de la pétrochimie, comme c'est le cas pour l'Isopropyl Palmitate. Des exemples d'esters huileux sont le caprylic capric triglyceride, le coco caprylate caprate, l'oleyl erucate, l'oleyl linoleate, le decyl oleate ou encore le PPG-3 benzyl ether myristate (identifiés par leur dénomination INCI).

On entend par huiles de silicones ou polysiloxanes, une huile comprenant au moins un atome de silicium, et notamment au moins un groupe Si-O. Comme huile de silicone, on peut notamment citer le phenylpropyldimethylsiloxysilicate, les dimethicones ou encore le cyclopentasiloxane (identifiés par leur dénomination INCI).

L'huile hydrocarbonée utilisée selon l'invention peut également être mise en mélange avec tout adjuvant ou additif habituellement utilisé dans les domaines pharmaceutiques. Bien entendu, l'homme du métier veillera à choisir le ou les éventuels additifs de la composition de manière telle que les propriétés avantageuses attachées ne soient pas ou substantiellement pas, altérées par l'addition envisagée. Parmi les adjuvants classiques susceptibles d'être contenus (selon le caractère hydrosoluble ou liposoluble de ces adjuvants), on peut citer notamment les tensioactifs moussants anioniques (tels que lauryl ether sulfate de sodium, alkyl phosphate de sodium, trideceth sulfate de sodium), amphotères (tels que alkyl bétaine, disodium cocoamphodiacetate) ou non ioniques de HLB supérieure à 10 (tels que POE/PPG/POE, Alkylpolyglucoside, polyglycery1-3hydroxylauryl ether) ; les conservateurs ; les séquestrants (EDTA) ; les antioxydants ; les parfums ; les matières colorantes telles que les colorants solubles, les pigments et les nacres ; les charges matifiantes, tenseurs, blanchissantes ou exfoliantes ; les filtres solaires ; les actifs cosmétiques ou dermatologiques et agents ayant pour effet d'améliorer les propriétés cosmétiques de la peau, hydrophiles ou lipophiles ; les électrolytes ; les polymères hydrophiles ou lipophiles, anioniques, non ioniques, cationiques ou amphotères, épaississants, gélifiants ou dispersants. Les quantités de ces différents adjuvants sont celles classiquement utilisées dans le domaine considéré, et par exemple de 0,01 à 20 % du poids total de la composition. Comme actifs utilisables avec l'huile hydrocarbonée, on peut citer par exemple, les vitamines hydrosolubles ou liposolubles comme la vitamine A (rétinol), la vitamine E (tocophérol), la vitamine C (acide ascorbique), la vitamine B5 (panthénol), la vitamine B3 (niacinamide), les dérivés de ces vitamines (notamment esters) et leurs mélanges ; le glutathion ; les antiseptiques ; les actifs antibactériens comme le 2,4,4'-trichloro-2'-hydroxy diphényl éther (ou triclosan), le 3,4,4'-trichlorocarbanilide (ou triclocarban) ; les antisébohrréïques ; les antimicrobiens tels que le peroxyde de benzoyle, la niacine (vit. PP) ; les amincissants tels que la caféine ; les azurants optiques, et tout actif approprié pour le but final de la composition, et leurs mélanges.

La composition pharmaceutique peut ainsi comprendre l'huile hydrocarbonée décrite précédemment à titre de principe actif médicamenteux, au moins un corps gras choisi parmi : les huiles végétales, les beurres végétaux, les éthers et alcools gras, les esters huileux, les alcanes et les huiles silicones et au moins un additif choisi parmi les additifs précités.

Cette composition pharmaceutique comprend un milieu physiologiquement acceptable, c'est-à-dire qui ne présente pas d'effets secondaires délétères et en particulier qui ne produit pas de rougeurs, d'échauffements, de tiraillements ou de picotements inacceptables pour un utilisateur. Ce milieu comprend éventuellement de l'eau et/ou au moins une huile en tant que corps gras, en plus de l'huile hydrocarbonée précitée.

Selon un mode de réalisation la composition pharmaceutique a une teneur en huile hydrocarbonée telle que décrite ci-dessus allant de 0,5 à 80%, de préférence de 1 à 50% et avantageusement de 5 à 30% en poids par rapport au poids total de la composition.

La composition pharmaceutique utilisée selon l'invention peut ainsi être une composition anhydre, une émulsion telle qu'une émulsion eau-dans-huile (E/H), une émulsion huile-dans-eau (H/E) ou une émulsion multiple (notamment E/H/E ou H/E/H), une nano-émulsion, ou encore une dispersion. La composition pharmaceutique utilisée selon l'invention peut se présenter sous forme de crème plus ou moins souple ou d'une émulsion vaporisable. Selon un mode de réalisation de l'invention, la composition pharmaceutique se présente sous la forme d'une émulsion eau-dans-huile (E/H) ou une émulsion multiple (notamment E/H/E ou H/E/H) ou une dispersion.

De préférence, l'invention ne couvre pas une émulsion à usage topique de type huile-dans-eau (E) comprenant pour 100% de sa masse :
- De 50% à 90% massique, d'une phase aqueuse (A) cosmétiquement acceptable,
- De 10% à 50% massique, d'une phase grasse (G) comprenant pour 100% de sa masse :
   - De 10% à 50% massique, plus particulièrement de 15% à 40% massique d'un mélange (M1) d'hydrocarbures saturés cycliques ou acycliques, linéaires ou ramifiés parmi lesquels au moins 95% massique comporte de quinze à dix-neuf atomes de carbone;
   - De 0,5% à 15% massique, d'au moins un agent tensioactif de type huile-dans-eau,
   - De 5% à 30% massique, d'au moins un agent de protection contre les rayonnements ultra-violets du soleil,
   - De 0% à 80% massique, d'au moins une huile et/ou une cire, étant entendu qu'une telle huile et/ou une telle cire ne répond pas à la définition du mélange (M1),
pour son utilisation dans une méthode de traitement thérapeutique destinée à prévenir et/ou traiter des maladies de la peau humaine liée à une exposition aux rayonnements ultra-violets du soleil, plus particulièrement les brûlures, les coups de soleil, les érythèmes, les cancers de la peau.

De préférence également, l'invention ne couvre pas l'utilisation d'une émulsion à usage topique de type huile-dans-eau (E) comprenant pour 100% de sa masse:
- de 40% à 90%, massique d'une phase aqueuse (A) cosmétiquement acceptable comprenant pour 100% de sa masse de 1% massique à 30% massique de glycérol, et
- de 10% à 60% massique d'une phase grasse (G) comprenant pour 100% de sa masse de 1% à 25% massique d'un mélange (M1) d'hydrocarbures saturés cycliques ou acycliques, linéaires ou ramifiés parmi lesquels au moins 95% massique comporte de quinze à dix-neuf atomes de carbone et de 0,5% à 15 % d'au moins un agent tensioactif de type huile-dans-eau,
en tant que produit de soin de la peau humaine.

L'huile hydrocarbonée ou la composition pharmaceutique peut également se présenter sous la forme de comprimés, de comprimés enrobés, de pelliculés, de granules, de gélules et capsules molles, de solutions, de sirops, d'émulsion, de suspension ou de mélange d'aérosol, de préférence sous la forme de gélules et capsules molles. Ainsi, l'huile hydrocarbonée peut être administrée par voie systémique, de préférence par voie orale. L'huile hydrocarbonée peut ainsi être destinée à être utilisée à titre de principe actif dans un alicament. L'homme du métier saura formuler un tel alicament selon ses connaissances générales. Par exemple, pour la production de comprimés, éventuellement enrobés et de capsule en gélatine dure, on pourra utiliser du lactose, de l'amidon de maïs ou ses dérivés, du talc, de l'acide stéarique ou ses sels, et pour la production de capsules en gélatine molle, on pourra utiliser des graisses, des cires, des polyols semi-solides ou liquides à titre de support.

L'huile hydrocarbonée ou la composition pharmaceutique la comprenant définie dans la présente invention se caractérise avantageusement par le fait qu'elle présente une stabilité d'une durée supérieure ou égale à 4 semaines, avantageusement supérieure ou égale à 6 semaines, la stabilité étant évaluée après un stockage sans agitation à température ambiante, à 40°C et à 50°C et correspondant à une évaluation visuelle de la coloration et de l'aspect ainsi qu'une évaluation olfactive et/ou une mesure de la viscosité.

Selon un mode de réalisation de l'invention, l'huile hydrocarbonée est le seul principe actif du médicament, que ce soit au sein d'une composition pharmaceutique topique ou d'un alicament. Bien entendu, dans un tel cas de figure, la composition pharmaceutique ou l'aliment comprendra généralement un support pharmaceutiquement acceptable.

L'huile hydrocarbonée définie dans la présente invention présente donc un effet antioxydant et/ou antiradicalaire et/ou anti-apoptotique et/ou anti-inflammatoire et/ou antibactérien et/ou antifongique. L'huile hydrocarbonée permet ainsi d'améliorer et/ou accélérer la cicatrisation.

L'huile hydrocarbonée utilisée selon l'invention peut également être en combinaison avec un ammonium quaternaire, par exemple parmi le chlorure de benzalkonium, le chlorure de didécyldiméthylammonium,..., de préférence, l'ammonium quaternaire est le chlorure de benzalkonium.

De préférence, l'ammonium quaternaire représente de 0,1 ppm à 40% en poids, de préférence de 0,5 ppm à 30% en poids, préférentiellement de 1 ppm à 20% en poids, encore plus préférentiellement de 3 ppm à 10% en poids, idéalement de 5 ppm à 1% en poids du poids total de la combinaison huile hydrocarbonée et ammonium quaternaire ou de la composition pharmaceutique comprenant ladite combinaison huile hydrocarbonée et ammonium quaternaire ou de l'alicament comprenant ladite combinaison huile hydrocarbonée et ammonium quaternaire.

L'invention décrit également une composition comprenant la combinaison de l'huile hydrocarbonée définie ci-dessus et d'un ammonium quaternaire tel que défini ci-dessus. Une telle composition peut comprendre :
- au moins 50% en poids, par rapport au poids total de la composition, d'une huile hydrocarbonée comprenant une teneur en poids d'isoparaffines allant de 90 à 100%, une teneur en poids de paraffines normales allant de 0 à 10% et présentant une teneur en carbone d'origine biologique supérieure ou égale à 90 % par rapport au poids total de l'huile hydrocarbonée ; et
- au moins un composé de type ammonium quaternaire.

Les composés de type ammonium quaternaire sont généralement utilisés comme conservateur dans les compositions cosmétiques, dermatologiques ou pharmaceutiques.

Selon un mode de réalisation, l'ammonium quaternaire mis en oeuvre dans la composition représente de 0,1 ppm à 40% en poids, de préférence de 0,5 ppm à 30% en poids, préférentiellement de 1 ppm à 20% en poids, encore plus préférentiellement de 3 ppm à 10% en poids, idéalement de 5 ppm à 1% en poids du poids total de la composition.

Selon un mode de réalisation, l'huile hydrocarbonée mise en oeuvre dans la composition représente au moins 60% en poids, de préférence au moins 70% en poids, préférentiellement au moins 80% en poids, encore plus préférentiellement au moins 90% en poids, idéalement au moins 99% en poids, du poids total de la composition.

Selon un mode de réalisation, la composition comprend une quantité majoritaire d'huile hydrocarbonée définie dans la présente invention et une quantité minoritaire d'ammonium quaternaire.

Par « quantité majoritaire », on entend une quantité d'au moins 50% en poids, par rapport au poids total de la composition.

Par « quantité minoritaire », on entend une quantité strictement inférieure à 50% en poids, par rapport au poids total de la composition.

Selon un mode de réalisation, la composition comprend au moins 60% en poids, de préférence au moins 70% en poids, préférentiellement au moins 80% en poids, encore plus préférentiellement au moins 90% en poids, idéalement au moins 99% en poids d'huile hydrocarbonée et moins de 40% en poids, de préférence moins de 30% en poids, préférentiellement moins de 20% en poids, encore plus préférentiellement moins de 10% en poids, idéalement moins de 1% en poids d'ammonium quaternaire, ledit ammonium quaternaire étant de préférence le chlorure de benzalkonium (BAC), les pourcentages étant exprimés par rapport au poids total de la composition.

### Procédé de traitement thérapeutique :

L'invention décrit également un procédé de traitement thérapeutique, ledit procédé comprenant une étape d'administration de l'huile hydrocarbonée telle que définie ci-dessus à titre de médicament.

L'huile hydrocarbonée peut être appliquée à travers une composition pharmaceutique telle que définie ci-dessus ou à travers un alicament tel que défini ci-dessus.

L'huile hydrocarbonée peut être administrée par voie topique sur la peau, le cuir chevelu, les cheveux ou des muqueuses, ou par voie orale. Dans le cas d'une application par voie topique, l'huile hydrocarbonée peut par exemple être appliquée sur une plaie ou une inflammation.

Le traitement thérapeutique permet notamment de réduire une inflammation, telle qu'une inflammation cutanée ou encore d'améliorer et/ou accélérer la cicatrisation.

Le procédé peut ainsi se caractériser par un effet antioxydant et/ou antiradicalaire et/ou anti-apoptotique et/ou anti-inflammatoire et/ou antibactérien et/ou antifongique.

### EXEMPLES

Dans la suite de la présente description, des exemples sont donnés à titre illustratif de la présente invention et ne visent en aucun cas à en limiter la portée.

### Huiles hydrocarbonées testées:

Le tableau 1 regroupe les propriétés physico chimiques des différentes huiles hydrocarbonées évaluées. Les huiles A et B sont des huiles hydrocarbonées utilisées selon l'invention.

**Tableau 1**

| Caractéristiques | Huile A | Huile B |
|---|---|---|
| Aromatiques (ppm) | <20 | <20 |
| Soufre (ppm) | 0.1 | 0.11 |
| % iso paraffines (w/w) | 98.9 | 96.2 |
| % n-paraffines (w/w) | 1.1 | 3.8 |
| % naphténiques (w/w) | 0 | 0 |
| C13 (iso) | 0.66 | 0 |
| C14 (iso) | 4.15 | 0 |
| C15 (iso) | 48.35 | 0 |
| C16 (iso) | 42.80 | 1.58 |
| C17 (iso) | 2.52 | 14.17 |
| C18 (iso) | 0.38 | 79.69 |
| C19 (iso) | 0 | 0.12 |
| C20 (iso) | 0 | 0.38 |
| C27 (iso) | 0 | 0.29 |
| Quantité de carbones d'origine biologique (%) | 97 | 98 |
| Point d'ébullition initial (°C) | 247.0 | 293.6 |
| Point ébullition 5% (°C) | 255.7 | 296.7 |
| Point ébullition 50% (°C) | 258.9 | 298.5 |
| Point ébullition 95% (°C) | 266.8 | 305.3 |
| Point d'ébullition final (°C) | 269.0 | 324.1 |
| Biodégradabilité OECD (28 jours) (%) | 80 | 83 |
| Indice de réfraction à 20°C | 1,4336 | 1,4394 |
| densité à 15°C (kg/m³) | 776,4 | 787,2 |
| Point éclair (°C) | 115 | 149 |
| Viscosité Cinématique à 40°C (cSt) | 2,49 | 3,87 |
| Pression de vapeur à 20°C (kPa) | <0,01 | <0,01 |
| Point Aniline (°C) | 93,2 | 99,5 |

Les normes et méthodes suivantes ont été utilisées pour mesurer les propriétés ci-dessus :
- point éclair : EN ISO 2719
- densité à 15°C : EN ISO 1185
- viscosité à 40°C : EN ISO 3104
- point aniline : EN ISO 2977
- Point d'ébullition : ASTM D86
- biodégradabilité : méthode OCDE 306
- indice de réfraction à 20°C : ASTM D 1218
- pression de vapeur : calculée selon des méthodes bien connues de l'homme du métier

### Essais sur les effets cellulaires

### Préparation des essais :

- Préparation des microplaques : 24 heures avant l'incubation avec les huiles A et B, les cellules HaCaT sont ensemencées à une densité cellulaire de 100 000 cellules/mL dans des microplaques 96 puits (Corning). Les microplaques sont ensuite placées à l'incubateur à 37°C. Dans une microplaque, chaque condition a été testée au minimum trois fois. Chaque expérience a été réalisée au minimum trois fois indépendamment afin de valider la reproductibilité des résultats et de réaliser des analyses statistiques.
- Incubation des cellules : deux durées d'incubation ont été sélectionnées pour les produits à tester purs : une incubation de quinze minutes et une incubation d'une heure. Après ces temps d'incubation, les microplaques sont vidées, les cellules sont rincées et incubées pendant 24 heures avec du milieu de culture enrichi à 2,5 % de SVF (Sérum de Veau Foetal) contenant 1% en poids de glutamine et 0,5% en poids d'antibiotiques.

### Analyse

Les effets cellulaires sont évalués directement sur cellules vivantes adhérentes par microtitration cytofluorimétrique (tests MiFALC-Microtitration Fluorimetric Assays on Live Cells). Le lecteur de microplaque utilisé dans l'étude est le cytomètre Safire (Tecan).

### Evaluation du stress oxydant :

Le stress oxydant correspond à un déséquilibre entre espèces oxydantes (principalement des espèces réactives de l'oxygène) et mécanismes de défenses antioxydants. Ce déséquilibre conduit à des effets délétères de type inflammation, dégénérescence cellulaire...

Le test H2DCF-DA (2',7'-diacétate de dihydrodichlorofluorescéine) est utilisé afin d'évaluer la production d'espèces réactives de l'oxygène. Cette molécule fluorogène entre dans les cellules par diffusion passive et s'accumule dans le cytosol où elle sera hydrolysée par les estérases cellulaires. En présence d'espèces réactives de l'oxygène, le produit formé peut être oxydé en un dérivé fluorescent. Ainsi, une augmentation de l'intensité de fluorescence est associée à une augmentation de la production d'espèces réactives (ERO) de l'oxygène et par extrapolation à une augmentation du stress oxydant dans les cellules étudiées.

La production d'espèces réactifs (ERO) exprimée en pourcentage par rapport au milieu de culture (MC) est indiquée dans le tableau 2 ci-dessous.

**Tableau 2 : Production d'espèces réactifs (% par rapport au MC)**

| | | MC | Huile A | Huile B |
|---|---|---|---|---|
| Production d'ERO | Incubation 15 min H2DCF-DA | 100 | 84* | 88 |
| | Incubation 1h H2DCF-DA | 100 | 73* | 100 |

| | | | | |
|---|---|---|---|---|
| *p<0,05; **p<0,01. | | | | |

Comme le montre le tableau 2, les huiles hydrocarbonées définies dans la présente invention n'induisent pas de surproduction d'espèces réactives de l'oxygène que ce soit après 15 minutes ou après 1 heure. Les huiles A et B diminuent la production d'espèces réactives de l'oxygène par rapport au témoin milieu de culture.

Ainsi, l'huile hydrocarbonée définie dans la présente invention peut être utilisée comme agent anti-oxydant.

### Evaluation de l'effet anti-dégénérescence cellulaire

### Evaluation de la condensation de la chromatine :

Pour ce test, les cellules HaCat sont incubées pendant une heure avec les huiles A ou B ou avec l'huile d'olive puis pendant 20 minutes avec le chlorure de benzalkonium (BAC), présent alors en une teneur de 8 ppm en poids. Le test HOECHST 33342 est réalisé afin d'évaluer la condensation de la chromatine.

Le chlorure de benzalkonium engendre un phénomène d'apoptose (mort cellulaire programmée). L'huile d'olive (HO) est une huile connue pour la protection cellulaire contre le chlorure de benzalkonium.

La mesure de la condensation de la chromatine exprimée en pourcentage par rapport au milieu de culture (MC) est indiquée dans le tableau 3 ci-dessous.

**Tableau 3 : Mesure de la condensation de la chromatine (% par rapport au MC)**

| | MC | MC + BAC | HO | HO+BAC | Huile A | Huile A + BAC | Huile B | Huile B + BAC |
|---|---|---|---|---|---|---|---|---|
| Mesure | 100 | 138*** | 96 | 112 | 91 | 100 | 86 | 94 |

| | | | | | | | | |
|---|---|---|---|---|---|---|---|---|
| ***p<0,001 | | | | | | | | |

Comme le montre le tableau 3, l'huile A et B présente un effet anti-apoptotique. En outre, on peut noter que l'effet apoptotique du chlorure de benzalkonium est grandement diminué en présence de l'huile hydrocarbonée A ou B, cette diminution étant bien supérieure à celle obtenue avec l'huile d'olive.

### Evaluation de l'activation du récepteur P2X7 :

Les récepteurs P2X7, présents dans de nombreux tissus, dont la peau, sont impliqués dans des phénomènes d'inflammation et de mort cellulaire, notamment par apoptose.

Le test YO-PRO-1 permet l'évaluation de l'activation des récepteurs P2X7. Cette sonde fluorescente entre dans les cellules uniquement si la perméabilité membranaire a été modifiée via l'activation des récepteurs P2X7 (ouverture de pores membranaires). La sonde utilisée peut alors se fixer sur l'ADN et émettre une fluorescence. Plus l'activation des récepteurs P2X7 est importante, plus l'intensité de la fluorescence est élevée.

L'activation du P2X7 exprimée en pourcentage par rapport au milieu de culture (MC) est indiquée dans le tableau 4.

**Tableau 4 : Activation du P2X7 (% par rapport au MC)**

| | | MC | Huile B |
|---|---|---|---|
| Activation du P2X7 | Incubation 15 min YO-PRO-1 | 100 | 109 |
| | Incubation 1h YO-PRO-1 | 100 | 135* |

| | | | |
|---|---|---|---|
| * p<0,05 | | | |

Comme le montre le tableau 4, l'huile hydrocarbonée B selon l'invention permet de diminuer l'activation du récepteur P2X7.

Ainsi, l'huile hydrocarbonée définie dans la présente invention présente un effet anti-apoptotique, bloquant le phénomène de mort cellulaire.

Ainsi, l'huile hydrocarbonée définie dans la présente invention peut par exemple être utilisée comme agent anti-apoptotique et anti-inflammatoire.

## Revendications

1. Huile hydrocarbonée destinée à être utilisée comme médicament, ladite huile hydrocarbonée comprenant une teneur en poids d'isoparaffines allant de 90 à 100%, une teneur en poids de paraffines normales allant de 0 à 10% et présentant une teneur en carbone d'origine biologique supérieure ou égale à 90 % par rapport au poids total de l'huile hydrocarbonée.

2. Huile hydrocarbonée destinée à être utilisée selon la revendication 1, en tant qu'antioxydant et/ou agent antiradicalaire et/ou agent anti-inflammatoire et/ou anti-apoptotique et/ou antibactérien et/ou antifongique.

3. Huile hydrocarbonée destinée à être utilisée selon la revendication 1 ou 2, dans le traitement des maladies de la peau telles que le psoriasis, les verrues et les cors, l'acné, les troubles de la séborrhée, l'herpès, les mycoses, l'eczéma, le zona et/ou des troubles de la cicatrisation.

4. Huile hydrocarbonée destinée à être utilisée selon l'une quelconque des revendications 1 à 3 pour une application topique.

5. Huile hydrocarbonée destinée à être utilisée selon l'une quelconque des revendications 1 à 3, pour une administration par voie systémique.

6. Huile hydrocarbonée destinée à être utilisée selon l'une quelconque des revendications précédentes dans laquelle l'huile hydrocarbonée comprend une teneur en poids d'isoparaffines allant de 95% à 100% et préférentiellement de 98% à 100% par rapport au poids total de l'huile hydrocarbonée.

7. Huile hydrocarbonée destinée à être utilisée selon l'une quelconque des revendications précédentes dans laquelle l'huile hydrocarbonée est choisie parmi les isoparaffines non-cycliques comprenant de 14 à 18 atomes de carbone.

8. Huile hydrocarbonée destinée à être utilisée selon l'une quelconque des revendications précédentes dans laquelle :
- l'huile hydrocarbonée comprend une teneur en poids de paraffines normales inférieure ou égale à 5% et préférentiellement inférieure ou égale à 2% par rapport au poids total de l'huile hydrocarbonée ; et/ou
- l'huile hydrocarbonée comprend une teneur en poids de composés naphténiques inférieure ou égale à 1%, de préférence inférieure ou égale à 0,5% et préférentiellement inférieure ou égale à 100 ppm par rapport au poids total de l'huile hydrocarbonée ; et/ou
- l'huile hydrocarbonée comprend une teneur en poids de composés aromatiques inférieure ou égale à 500 ppm, de préférence inférieure ou égale à 300 ppm, préférentiellement inférieure ou égale à 100 ppm, plus préférentiellement inférieure ou égale à 50 ppm et avantageusement inférieure ou égale à 20 ppm par rapport au poids total de l'huile hydrocarbonée.

9. Huile hydrocarbonée destinée à être utilisée selon l'une quelconque des revendications précédentes dans laquelle :
- l'huile hydrocarbonée a une température d'ébullition allant de 230 à 340°C, de préférence de 235 à 330°C et plus préférentiellement de 240 à 325°C selon la norme ASTM D86 ; et/ou
- l'huile hydrocarbonée a un point éclair supérieur ou égal à 110°C selon la norme EN ISO 2719 ; et/ou
- l'huile hydrocarbonée a une pression de vapeur à 20°C inférieure ou égale à 0,01kPa.

10. Huile hydrocarbonée destinée à être utilisée selon l'une quelconque des revendications précédentes dans laquelle l'huile hydrocarbonée est obtenue par un procédé d'hydrogénation catalytique à une température de 80 à 180°C et à une pression de 50 à 160 bars d'une charge d'origine biologique désoxygénée et/ou isomérisée.

11. Huile hydrocarbonée destinée à être utilisée selon l'une quelconque des revendications précédentes, se présentant sous la forme d'une composition pharmaceutique comprenant au moins une huile hydrocarbonée telle que définie dans l'une des revendications 1 à 10, de préférence en une quantité allant de 0,5 à 80%, préférentiellement de 1 à 50% et avantageusement de 5 à 30% en poids par rapport au poids total de la composition pharmaceutique.

12. Huile hydrocarbonée destinée à être utilisée selon la revendication 11, dans laquelle la composition pharmaceutique comprend au moins un corps gras choisi parmi : les huiles végétales, les huiles hydrocarbonées autres que l'huile hydrocarbonée définie dans les revendications 1 à 10, les beurres végétaux, les éthers et alcools gras, les esters huileux, les alcanes et les huiles silicones et/ou au moins un additif.

13. Huile hydrocarbonée destinée à être utilisée selon la revendication 11 ou 12, dans laquelle la composition pharmaceutique est une composition anhydre, une émulsion telle qu'une émulsion eau-dans-huile (E/H), une émulsion huile-dans-eau (H/E) ou une émulsion multiple (notamment E/H/E ou H/E/H), une nano-émulsion, ou encore une dispersion ou un gel.

14. Huile hydrocarbonée destinée à être utilisée selon l'une quelconque des revendications 1 à 10, se présentant sous la forme d'un alicament comprenant au moins une huile hydrocarbonée telle que définie dans l'une des revendications 1 à 10, de préférence en une quantité allant de 0,5 à 80%, préférentiellement de 1 à 50% et avantageusement de 5 à 30% en poids par rapport au poids total de l'alicament.

15. Huile hydrocarbonée destinée à être utilisée selon l'une quelconque des revendications 1 à 14, en combinaison avec un ammonium quaternaire.

16. Huile hydrocarbonée destinée à être utilisée selon la revendication 15, dans laquelle l'ammonium quaternaire est choisi parmi le chlorure de benzalkonium et le chlorure de didécyldiméthylammonium, seuls ou en mélange.

17. Huile hydrocarbonée destinée à être utilisée selon la revendication 15 ou 16, dans laquelle l'ammonium quaternaire représente de 0,1 ppm à 40% en poids, de préférence de 0,5 ppm à 30% en poids, préférentiellement de 1 ppm à 20% en poids, encore plus préférentiellement de 3 ppm à 10% en poids, idéalement de 5 ppm à 1% en poids du poids total de la combinaison huile hydrocarbonée et ammonium quaternaire ou de la composition pharmaceutique ou de l'alicament.
